# EUROPEAN PATENT APPLICATION

(11) **EP 1 621 249 A1**
(43) Date of publication of application: **01.02.2006**
(21) Application number: 04713635.3
(22) Date of filing: 23.02.2004
(51) Int. Cl.: B01J 19/08, B01J 19/00, C07K 1/02

(54) **CHEMICAL PROCESSES BY THE USE OF COMBINATIONS OF SOLVENTS CAPABLE OF TAKING REVERSIBLY HOMOGENEOUSLY MIXED STATE AND SEPARATED STATE DEPENDENTLY ON TEMPERATURE**

(30) Priority: 24.02.2003 JP 2003045815
(71) Applicant: Tokyo University of Agriculture and Technology Tlo Co., Ltd., Tokyo 184-8588 (JP)
(72) Inventor: CHIBA, Kazuhiro, Tokyo Univ. of Agriculture & Tech, Fuchu-shi, Tokyo 183-8509 (JP)
(74) Representative: Lang, Johannes
(86) International application number: PCT/JP2004/002072
(87) International publication number: WO 2004/073852

(57) **Abstract**

(Purpose)

Providing a solvent set preferable for use as miscible-multiphase organic solvent system. While the miscible temperature of a solvent system is dropped by using a mixture solvent of plural solvents in the related art, the temperature can be dropped by a single solvent. Providing a solvent set appropriate for chemical processes imparted electric energy, such as electrochemical process.

(Means for Resolution)

Use of a chemical process using a combination of a first solvent and a second solvent, where the miscible state and the separation state reversibly change depending on the temperature, where the first solvent includes an alkane-series compound and/or a cycloalkane-series compound; and the second solvent includes at least one selected from the group consisting of a carbonate (carboxylic acid ester) compound, a carbamate (urethane) compound, an urea compound, a cyclic amide compound and a cyclic ketoamine compound.

## Description

### Field of the Invention

The invention provides a combination of solvents preferable for use in "miscible-multiphase organic solvent system", particularly a combination of solvents suitable for peptide synthesis process and chemical processes giving electric energy, such as electrochemical process. The invention relates to a technique greatly improving the efficiency of such chemical processes. The term "combination of solvents" is referred to as "solvent set" hereinbelow.

The solvent set is a combination of a first solvent with a relatively low polarity and a second solvent with a relatively high polarity. These first and second solvents may individually be a mixture solvent of plural solvents. It is needless to say that these solvents may individually be a single solvent satisfactorily.

### Related Art

The present inventor proposed a novel solvent set capable of readily controlling the state change between miscible state and separation state depending on the temperature and thereby capable of constructing a chemical process readily achieving reaction control and the separation and purification of reaction products and the like via the control of the state change (patent reference 1).

One example of the use of the solvent set is peptide synthesis reactions in liquid phase, which surpass but are never inferior to the solid phase peptide synthesis in the related art (see non-patent reference 1 and patent reference 2).

Another application example thereof is selective oxidation process with the solvent set (see patent reference 1, Paragraph Nos. 0024 to 0025).

### <DMI>

Meanwhile, a known compound "dimethylimidazolidinone: 1,3-dimethyl-2-imidazolidinone" (referred to as "DMI" hereinbelow) is an industrially useful solvent. DMI is a nitrogen-containing five-membered cyclic compound and is a solvent with a high boiling point, as developed by Mitsui Chemical Co., Ltd. (Mitsui To-atsu Chemical Co., Ltd. under the old name) and Kawaken Fine Chemical Co. , Ltd. in cooperation. DMI has the following great properties described below in 1 to 4. DMI is used in a wide variety of utilities.
1. DMI is one of cyclic urea compounds and is a colorless, transparent, non-protonic polar solvent with a high polarity.
2. DMI is stable even in the presence of acids and alkalis, compared with general non-protonic polar solvents and has great alkali resistance and acid resistance even at high temperature, in particular.
3. DMI has readily handleable properties such as the high boiling point and the high flash point but the low freezing point. (Boiling point: 225.5°C; flash point: 120°C; freezing point: 8.2°C)
4. DMI has strong dissolution potencies for various inorganic and organic compounds. Due to the "high dielectric constant" and the "solvation effect", DMI effectively promotes reactions but suppresses side reactions.

Typical utilities thereof are 1) solvents for the synthesis of products with high added values, such as pharmaceutical products, agricultural chemicals, dyes and pigments; 2) rinsing agents of electronic parts and molds; and 3) solvents for the polymerization of polymer compounds (see patent references 3, 4 and 5).

### <First solvent>

The patent reference 1 includes the following descriptions. The first solvent is basically an organic solvent with a low polarity. A compound group composing the solvent includes alkane, cycloalkane, alkene, alkyne and aromatic compounds. Among them, cycloalkane-series compounds are preferable and "cyclohexane" is particularly preferable. Probably, the preference has a relation with the occurrence of the conversion between cyclohexane isomers of chair and boat conformations under relatively mild conditions in temperature in view of the other solvent. Cyclohexane has a relatively high melting point of 6.5°C, from which products can be solidified and separated after reaction, advantageously. Even at the recovery step as the final step, cyclohexane is advantageous preferably from the standpoint.

### <Second solvent>

The same patent reference 1 includes the following descriptions. An organic solvent composing the other solvent or a mixture solvent (second solvent) in combination with the first solvent is basically an organic solvent with a high polarity. Preferably, the second solvent is composed of at least one selected from the group consisting of nitroalkane, nitrile, alcohol, halogenated alkyl, amide compounds and sulfoxide.

Further, the second solvent is specifically nitroalkane with an alkyl group with 1, 2 or 3 carbon atoms, nitrile with an alkyl group with 1,2 or 3 carbon atoms, amide compounds where the alkyl group and acyl group or formyl group in N-dialkyl or N-monoalkylamide has carbon atoms of 6 or less in total, alcohol with 8 or less carbon atoms, sulfoxide with an alkyl group with 1, 2 or 3 carbon atoms, or halogenated alkyl with an alkyl group with 6 or less carbon atoms.

### <Miscible temperature (separation temperature)>

The patent reference 1 describes that the modification of the composition of the first solvent or the second solvent can lead to the free modification of the temperature at which miscible state and phase separation state switch to each other. A figure in the patent reference 1 discloses experimental data about the composition of a mixture solvent of the first solvent cyclohexane (CH) and the second solvent nitroalkane (NA) and about the change of the miscible temperature.

Specifically in the figure, volume ratios of CH and NA are preset at 1:5, 2:5, 1 : 1 and 5 : 1 as parameter; the volume mix ratio of nitromethane (NM) and nitroethane (NE) composing each NA type is expressed on crosswise axis, while solvent temperature is on longitudinal axis; and the miscible temperature data is plotted in the figure, when the two solvents are mixed together.

Additionally in another figure, cyclohexane (CH) as the first solvent and a second solvent are fixed at equal 1:1 volumes (individually at 50 % by volume) ; the second solvent is a mixture solvent of nitromethane (NM) and nitroethane (NE) or a mixture solvent of acetonitrile (AN) and propionitrile (PN) or a mixture solvent of dimethylformamide (DMF) and dimethylacetamide (DMA); then, the volume mix ratio of the second solvent is expressed on crosswise axis, while the solvent temperature is expressed on longitudinal axis; and the miscible temperature data of the two solvents when mixed together is plotted in the figure.

The figures in the patent reference 1 indicate that the miscible temperature (separation temperature) changes at a temperature of 20°C to 60°C, depending on the compositions of the first and second solvents. In other words, the miscible temperature (separation temperature) of the solvent set of the first and second solvents can appropriately be changed within a range of 20°C to 60°C, by a means for modifying the compositions of the first and second solvents.

### <Chemical process using solvent set>

As described in the patent reference 1, the chemical process using such solvent set is not limited to any specific process but is applicable to liquid phase peptide synthesis process of sequentially adding amino acids as described in the patent reference 2 or general electrochemical reaction processes.

So as to promote chemical reaction, further, the chemical process may be a process imparted electric energy with no specific limitation to electrochemical reaction process. As one example thereof, the application thereof to the Diels-Alder reaction process is described (see patent reference 1, Paragraph Nos. 0026 to 0027).

### <Polarity or dielectric constant>

As to polarity and dielectric constant, generally, non-patent reference 2 and non-patent reference 3 describe technical standards. Specifically, polarity (ET (30)) can be experimentally evaluated according to the method described in the non-patent reference 3, while dielectric constant is experimentally evaluated according to the method described in the non-patent reference 2.

According to the standards, the conditions of solvents with high polarity are described as follows: the polarity (ET (30)) is 25 or more or the dielectric constant is 20 or more. According to the standards, the conditions of solvents with low polarity are described as follows: the dielectric constant is 0 to 15 or the polarity (Et (30)) is less than 20.
(Patent reference 1)
   Japanese Patent Application 2001-254109 entitled "Miscible-multiphase organic solvent system".
(Patent reference 2)
   Japanese Patent Application 2001-385493 entitled "Liquid phase peptide synthesis process of sequentially adding amino acids with miscible-multiphase organic solvent system".
(Patent reference 3)
   Laid-open official gazette of JP-A-54-144348
(Patent reference 4)
   Laid-open official gazette of JP-A-63-108027
(Patent reference 5)
   Laid-open official gazette of JP-A-05-170713
(Non-patent reference 1)
   "A liquid-phase peptide synthesis in cyclohexane-based biphasic thermomorphic systems", Kazuhiro Chiba, Yusuke Kono, Shokaku Kim, Kohsuke Nishimoto, Yoshikazu Kitano and Masahiro Tada, Chem. Commun., 2002, (Advance Article), The Royal Society of Chemistry, 1766-1767, 2002, (First published on the web 15^{th} July, 2002)
(Non-patent reference 2)
   J.A. Riddick and W. B.Bunger (eds.), Organic Solvents, Vol. II of Techniques of Organic Chemistry, Third Edition, Wiley-Interscience, New York, 1970.
(Non-patent reference 3)
   C. Reichardt and K. Dimroth, Fortshr. Chem. Forsch. 11, 1 (1968) , C. Reichardt, Justus Liebigs Ann. Chem. 725, 64 (1971).

### Disclosure of the Invention

It is an object of the invention to provide a solvent set preferable for use as "miscible-multiphase organic solvent system". Particularly, it is an object of the invention to allow the miscible temperature of a solvent system to drop by using a single solvent, in contrast to the use of a mixture solvent of plural solvents in the related art to drop the miscible temperature of a solvent system.

Additionally, it is an additional object of the invention to provide a solvent set suitable for a chemical process imparted an electric energy, such as electrochemical process.

The invention of this application relates to a chemical process using a solvent set of a first solvent and a second solvent, where the miscible state and the separation state change reversibly depending on the temperature. The inventor examined and found a second solvent preferable for the combination with an alkane-series compound or a cycloalkane-series compound as the first solvent (or the main component of the first solvent) for achieving the objects.

Specifically, the inventor found that the second solvent for the combination with the first solvent was a carbonate (carboxylic acid ester) compound or a carbamate (urethane) compound or an urea compound or a cyclic amide or a cyclic ketoamine, or the main component of the second solvent was a carbonate (carboxylic acid ester) compound or a carbamate (urethane) compound or an urea compound or a cyclic amide or a cyclic ketoamine.

Particularly preferably, the carbonate (carboxylic acid ester) compound or the carbamate (urethane) compound or the urea compound is a cyclic carbonate (carboxylic acid ester) compound or a cyclic carbamate (urethane) compound or a cyclic urea compound.

Still more preferably, the cyclic urea compound is dialkylimidazolidinone, where the total carbon atoms of the two alkyl groups are 2 or more (the carbon atoms in total are 5 or more). Herein, no specific upper limit of the total carbon atoms of the cyclic urea compound exists. However, a cyclic urea compound with 30 or less carbon atoms in total is generally used. The second solvent is most preferably DMI (dimethylimidazolidinone).

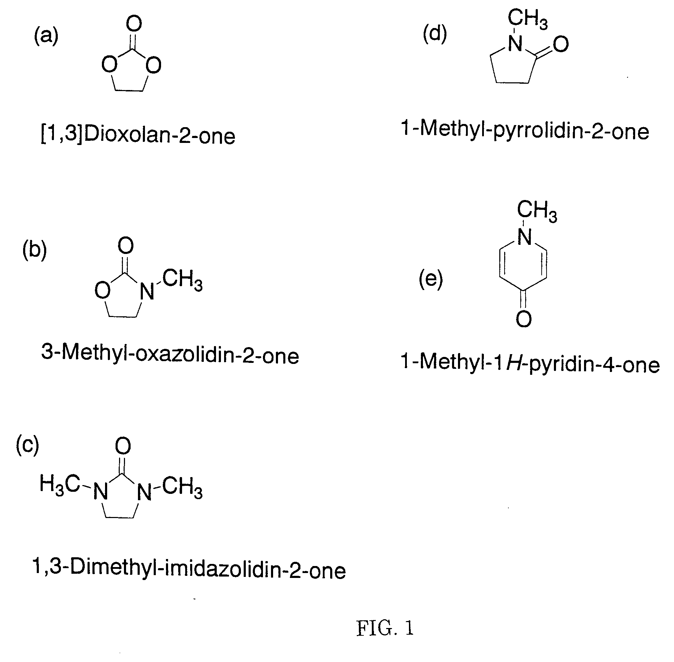

Fig. 1 shows an example of the cyclic carbonate (carboxylic acid ester) compound (Fig.1 (a)), an example of the cyclic carbamate (urethane) compound (Fig.1 (b)) , the most preferable example DMI (dimethylimidazolidinone) of the cyclic urea compound as the most preferable example (Fig. 1 (c)) , the most preferable example of the cyclic amide (Fig .1 (d) ) and the most preferable example of the cyclic ketoamine (Fig.1 (e)).

A great number of industrial utilities of DMI have been found as described in the section "Description of the Related Art". The use of DMI as a second solvent in a solvent set is novel and involves a unique effect.

Furthermore, the same effect is found in not only DMI but also general carbonate (carboxylic acid ester) compounds or carbamate (urethane) compounds or urea compounds or cyclic amides or cyclic ketoamines.

The effect can be said as follows. In the related art, a mixture solvent of plural solvents is used to drop the miscible temperature (separation temperature) of a solvent system. However, this drop can be attained by a single solvent, for example DMI alone.

### Brief Description of the Drawing

Fig. 1 shows an example of the cyclic carbonate (carboxylic acid ester) compound (a), an example of the cyclic carbamate (urethane) compound (b), a cyclic urea compound DMI (dimethylimidazolidinone) as the most preferable example (c), an example of the cyclic amide (d) and an example of the cyclic ketoamine (e).

### Best Mode for Carrying Out the Invention

The effect is now described below in experiments using DMI. The effect is also exerted, using other carbonate (carboxylic acid ester) compounds, carbamate (urethane) compounds, urea compounds, cyclic amides or cyclic ketoamines. Therefore, the description is not shown here.

### Experiment 1: Miscible-biphasic separation depending on the temperature of cyclohexane and DMI

When 10 ml of cyclohexane as a first solvent and 10ml of dimethylimidazolidinone (DMI) as a second solvent are mixed together at 20°C, a solution in biphasic separation is prepared. When the solution is gradually heated, the two phases are miscible with each other around 30°C. At the temperature or more, then, a completely homogenous miscible state is formed. When the homogenous solution is again cooled to 25°C, phase separation immediately occurs, so that a solution in biphasic separation is prepared. The phenomenon is reversible by heating and cooling. The miscible temperature (separation temperature) of the solvent set is 25°C.

### Experiment 2: Variable control of miscible-biphasic separation temperature using a cycloalkane mixture as first solvent (second solvent is DMI)

A mixture of cyclohexane and cyclooctane is used as a first solvent in contrast with Experiment 1. By changing the mix ratio, the miscible temperature can be controlled at an appropriate temperature from 30°C to 38°C.

A mixture of cyclohexane and cyclopentane is used as a first solvent in contrast with Experiment 1. By changing the mix ratio, the miscible temperature can be controlled at an appropriate temperature from 11°C to 30°C.

### Experiment 3: Variable control of miscible-biphasic separation temperature using a mixture of cycloalkane and alkane as first solvent (second solvent is DMI)

A mixture of cyclohexane and n-octane is used as a first solvent in contrast with Experiment 1. By changing the mix ratio, the miscible temperature can be controlled at an appropriate temperature from 30°C to 65°C.

A mixture of methylcyclohexane and n-octane is used as a first solvent in contrast with Experiment 1. By changing the mix ratio, the miscible temperature can be controlled at an appropriate temperature from 30°C to 65°C.

A mixture of cyclohexane and n-tetradecane is used as a first solvent in contrast with Experiment 1. By changing the mix ratio, the miscible temperature can be controlled at an appropriate temperature from 30°C to 95°C.

A mixture of decalin and n-tetradecane is used as a first solvent in contrast with Experiment 1. By changing the mix ratio, the miscible temperature can be controlled at an appropriate temperature from 0°C to 95°C.

### Experiment 4: Variable control of miscible-biphasic separation temperature by mixing a polar solvent in second solvent (first solvent is cyclohexane)

A mixture of DMF and DMI is used as a second solvent in contrast with Experiment 1. By changing the mix ratio, the miscible temperature can be controlled at an appropriate temperature from 30°C to 45°C.

By using DMI alone as the second solvent as in the Experiments 1 through 3, the miscible temperature (separation temperature) of the solvent systems could sufficiently be dropped. This is advantageous for peptide synthesis. It is needless to say that the miscible temperature (separation temperature) may satisfactorily be adjusted with a mixture solvent with the main component DMI as in the Experiment 4. In other words, carbonate (carboxylic acid ester) compounds; carbamate (urethane) compounds, urea compounds, cyclic amides or cyclic ketoamines typically including DMI are widely applicable as a second solvent of such solvent set.

Herein, the claims are now additionally described. The chemical process according to claim 1 encompasses intramolecular and intermolecular reactions, intramolecular and intermolecular interactions, separation based on the difference in electron transfer velocity or substance transfer velocity, extraction and separation based on the difference in partition coefficients, and solvent fractionation.

Additionally, the chemical process also encompasses peptide synthesis process.

Particularly, the solvent set of the invention is applicable to a chemical process imparted electric energy, such as electrochemical process . In other words, the solvent system of the invention is characteristically applicable to electrochemical reaction because electrolysis is possible when an electrolyte (namely a salt) is dissolved in a second solvent with a high polarity.

As described above, apparently, the solvent set can be used for a wide variety of chemical reactions, with no limitation to electrochemical reaction. One specific example thereof is Diels-Alder reaction (see patent reference 1, Paragraph Nos. 0026 to 0027).

A solvent system with "a solvent suitable as an electrolysis solution" as a second solvent is satisfactory as the construction applicable to such wide variety of chemical reactions.

When this construction is represented in terms of polarity or dielectric constant, satisfactorily, the dielectric constant of the second solvent is 20 or more or the polarity (ET (30)) of the second solvent is 25 or more. This indicates that the dielectric constant of the first solvent is 0 to 15 or the polarity (ET (30)) of the first solvent is less than 20, satisfactorily.

Examples of the chemical process are now described below. One of the solvent set in the Examples has a dielectric constant of 20 or more or a polarity (ET (30)) of 25 or more, while the other thereof has a dielectric constant of 0 to 15 or a polarity (ET (30)) for the second solvent of less than 20.

### Example 1

### <Selective oxidation reaction using solvent system containing electrolyte and oxidizable organic compound>

A mix solution of cyclohexane and DMI was prepared at 20°C and one atmospheric pressure. Then, the organic solvent system was separated in two phases. 200 mg of lithium perchlorate as a support electrolyte and 10 mg of hexadecane thiol as an electrolysis substrate were added to the mix solution.

Grassie carbon electrode (action electrode), platinum cathode, and silver silver chloride standard electrode were inserted in the lower solution layer (containing DMI as the main component and dissolving mainly lithium perchlorate therein). Then, the electric potential was reciprocally changed from -0.2 V to 2.0 V at 100 mV/second (cyclic voltammetry) , to measure the electric current correspondingly.

Because the solvent system was at a state of separated solvents at the temperature, consequently, almost no oxidation of hexadecane thiol occurred, with no significant observed peak representing the oxidation of thiol group.

When the solution was heated to 30°C, then, the system wholly turned a homogenous solution (homogenous miscible mix solution system).

The voltage-electric current curve was measured at that state. A signal very significantly representing the oxidation of thiol group was observed. Further, the voltage-electric current curve was again measured after cooling to 20°C. However, the oxidation wave was not observed any more.

These indicate that no electron transfer occurred on the electrode surface because most of the support electrolyte was dissolved in the lower layer (the nitroalkane phase) at the state of the solution system in phase separation at 20°C (separated solvents) and most of the electrolysis substrate was dissolved in the upper layer (cyclohexane layer) at that state. After the solution system changed to the homogenous miscible mix solution system, the support electrolyte and the electrolysis substrate were dissolved in the homogenous solution. Therefore, discharge onto the electrode readily occurred. By controlling slight temperature change in such manner, the distribution state of the electrolyte and the solute in the solvent system can be changed, to control the progress and selectivity of chemical reaction.

### Example 2

### <Electrolytic Diels-Alder reaction of octadecyl 2,5-dihydroxybenzoate and 2,3-dimethylbutadiene>

12 mg of octadecyl 2,5-dihydroxybenzoate and 30 mg of 2,3-dimethylbutadiene are dissolved in 5 ml of cyclohexane, to which 5 ml of DMI and 50 mg of acetic acid are further added. The resulting solution was in biphasic separation at 20°C and one atmospheric pressure. When the solution was heated to 30°C (one atmospheric pressure), the resulting solution turned a homogenous solution. At that state, an electricity level corresponding to 2.2 electrons per one benzoate molecule was given at a 2.0-V terminal voltage and an electric current of 0.3 mA, using Grassie carbon plate as the anode and a platinum plate as the cathode. Subsequently, the reaction solution was cooled to 25°C, for phase separation, to recover the product from the cyclohexane phase. Yield of 48 %.

### Example 3

### <Reaction for forming peptide bond, using cyclic urea compound as second solvent>

1 mmol of 2-amino-3-methyl-butyric acid 3,4,5-tris-octadecyloxy-benzyl ester was dissolved in 100 ml of cyclohexane. 50 ml of a DMI solution containing 3 mmol of Fmoc-Gly-OBt and 5 mmol of diisopropylcarbodiimide (DIPCD) was added to the resulting solution, for agitation for 90 minutes. Then, the reaction system was cooled to 0°C under agitation. After cooling, the DMI solution was removed. The resulting cyclohexane phase was rinsed three times with 200 ml of DMI. From the cyclohexane solution, 2-[2-(9H-fluorein-9-yl-methoxycarbonylamino)-acetylamino]-3-methyl-butyric acid 3,4,5-tris-octadecyloxy-benzyl ester was recovered at a yield of 97%.
¹H-NMR(400MHz)δ:7.77(2H,d,J=7.3Hz),7.59(2H,d,J=7.3Hz),7.40( 2H,t,J=7.3Hz),7.31(2H,dt,J=0.7,7.3Hz),6.52(2H,s),6.38(1H,d ,J=8.4Hz),5.44-5.37(1H,br),5.10(1H,d,J=12.1Hz),5.02(1H,d,J =12.1Hz),4.62(2H,dd,J=8.4,4.8Hz),4.42(2H,d,J=7.0Hz),4.24(1 H,t,J=7.0Hz),3.96-3.92(8H,m),2.21-2.16(1H,m),1.81-1.76(4H, m),1.75-1.70(2H,m),1.48-1.43(6H,m),1.37-1.21(84H,br),0.91( 3H,d,J=7.0Hz),0.88(9H,t,J=7.0Hz),0.86(3H,d,J=7.0Hz);¹³C-NMR (150MHz)δ:171.5,168.7,156.5,153.1,143.6,141.2,138.3,130.0,1 27.7,127.0,125.0,120.0,107.0,73.4,69.2,67.5,67.4,57.1,47.1 ,32.0,31.4,30.4,29.8,29.7,29.5,29.4,26.1,22.8,19.0,17.7,14 . 2 ; MALDITOF-MS (pos) calcdforC₈₃H₁₃₈N₂O₈[M+Na]⁺1314 , found1314 .

### Example 4

### <Reaction for forming peptide bond, using cyclic amide compound as second solvent>

1 mmol of 2-amino-3-methyl-butyric acid 3,4,5-tris-octadecyloxy-benzyl ester was dissolved in 100 ml of methylcyclohexane. 50 ml of N-methyl-2-pyrrolidone (NMP, 1-methyl-pyrrolidin-2-one) solution containing 3 mmol of Fmoc-Gly-OBt and 5 mmol of diisopropylcarbodiimide (DIPCD) was added to the resulting solution, for agitation at 20°C for 90 minutes. Then, the reaction system was cooled to -10°C under agitation. The solute components were partially deposited. Then, the solution was heated gradually to 10°C, to dissolve the deposited components. The solution then was in biphasic separation. The NMP solution as the lower layer was removed, while the methylcyclohexane phase was rinsed three times with 200 ml of NMP. From the methylcyclohexane solution, 2-[2-(9H-fluorein-9-yl-methoxycarbonylamino)-acetylamino]-3-methyl-butyric acid 3,4,5-tris-octadecyloxy-benzyl ester was recovered at a yield of 94 %.

### Industrial Applicability

The solvent set of the invention can be applied to a wide range of chemical processes including intramolecular and intermolecular reactions, intramolecular and intermolecular interactions, separation based on the difference in electron transfer velocity or substance transfer velocity, extraction and separation based on the difference in partition coefficients, and solvent fractionation.

While the miscible temperature of a solvent system is dropped using a mixture solvent of plural solvents in the related art, the temperature can be dropped by a single solvent. Any hardware or software for controlling the mix composition of plural solvents in the process is not needed any more in such manner. Accordingly, the solvent set is very advantageous industrially.

Additionally, a solvent combination particularly appropriate for chemical processes imparted electric energy, such as electrochemical process is provided. Therefore, the efficiency of such chemical processes can effectively be improved greatly.

## Claims

1. A chemical process using a combination of a first solvent and a second solvent, where the miscible state and the separation state reversibly change depending on the temperature,
where the first solvent includes an alkane-series compound and/or a cycloalkane-series compound; and
the second solvent includes at least one selected from the group consisting of a carbonate (carboxylic acid ester) compound, a carbamate (urethane) compound, an urea compound, a cyclic amide compound and a cyclic ketoamine compound.

2. The chemical process according to claim 1, where the dielectric constant of the first solvent is 0 to 15 and/or the polarity of the first solvent (ET (30)) is less than 20, and
where the dielectric constant of the second solvent is 20 or more and/or the polarity of the second solvent (ET (30)) is 25 or more.

3. The chemical process according to claim 1 or 2, where the carbonate (carboxylic acid ester) compound, the carbamate (urethane) compound and the urea compound are a cyclic carbonate (carboxylic acid ester) compound, a cyclic carbamate (urethane) compound and a cyclic urea compound, respectively.

4. The chemical process according to claim 3, where the cyclic urea compound is dialkylimidazolidinone with 5 or more carbon atoms in total.

5. The chemical process according to claim 1, where the cyclic amide compound or the cyclic ketoamine compound includes 10 or less carbon atoms in total.

6. The chemical process according to any one of claims 1 through 5, the chemical process including peptide synthesis process.
